# DEMANDE DE BREVET EUROPEEN

(11) **EP 3 915 586 A1**
(43) Date de publication de la demande: **01.12.2021**
(21) Numéro de dépôt: 20215726.9
(22) Date de dépôt: 07.10.2010
(51) Int. Cl.: A61K 47/00, A61K 39/12, A61K 39/205, A61K 9/00, A61K 47/18, A61K 47/26, A61K 39/00, A61P 31/12, A61P 31/14

(54) **EXCIPIENT STABILISANT POUR VACCIN A VIRUS ENTIERS INACTIVES**

(30) Priorité: 07.10.2009 FR 0904795
(62) Demande divisionnaire de: 10781953.4
(71) Demandeur: Sanofi Pasteur, 69007 Lyon (FR)
(72) Inventeur: FRANCON, Alain, 69690 Bessenay (FR); CHEVALIER, Michel, 38270 Beaurepaire (FR); MORENO, Nadège, 69290 Craponne (FR); CALVOSA, Eric, 69610 Haute Rivoire (FR); CIGARINI, Sandrine, 69290 Saint Genis les Ollières (FR); FABRE, Virginie, 06650 Le Rouret (FR)
(74) Mandataire: Ernest Gutmann - Yves Plasseraud S.A.S.

(57) **Abrégé**

L'invention a pour objet une composition vaccinale comprenant :
a) du virus entier inactivé et,
b) un excipient stabilisant qui comprend :
i. une solution tampon,
ii. un mélange d'acides aminés essentiels et non essentiels,
iii. un disaccharide,
iv. un polyol,
v. un agent chélatant,
vi. de l'urée ou un dérivé de l'urée, et
vii. un tensioactif non ionique.

## Description

La présente invention a pour objet une composition vaccinale comprenant du virus entier inactivé et un excipient qui stabilise la composition vaccinale, la composition de l'excipient comprenant une solution tampon, un mélange d'acides aminés essentiels et non essentiels, un disaccharide, un polyol, un agent chélatant, de l'urée ou un dérivé de l'urée et un tensioactif non ionique. Elle concerne également un procédé de préparation de cette composition vaccinale ainsi que la composition de l'excipent en tant que telle.

Dans le domaine des vaccins viraux, on distingue classiquement 3 types de vaccins viraux : les vaccins à virus vivants, les vaccins à virus entiers inactivés et les vaccins viraux sous unitaires. Ces 3 types de vaccins se distinguent par des caractéristiques qui leur sont propres et qui conditionnent la composition des excipients qui sont utilisés pour leur conservation. Les vaccins sous unitaires contenant un nombre restreint d'antigènes viraux sont généralement plus faciles à conserver que les vaccins à virus entiers inactivés pour lesquels on cherche à conserver l'intégrité structurelle des virus même s'ils sont tués, ou que les vaccins à virus vivants pour lesquels on cherche à conserver également le pouvoir infectieux des virus. Pour chaque type de vaccins viraux des excipients adaptés ont été préparés. Pour les vaccins viraux à virus atténués différents excipients stabilisants ont été préparés en fonction de la préparation de virus atténués à conserver. JP 57007423 décrit un excipient à base de disaccharide et de polyalcool dans un tampon phosphate pour stabiliser une souche atténuée du virus de la rougeole. EP 065905 décrit un excipient comprenant un ou plusieurs acides aminés choisis parmi un groupe de onze acides aminés, du lactose et du sorbitol dans un tampon phosphate pour stabiliser une souche atténuée du virus de la dengue. Enfin EP 0869814 décrit une composition vaccinale qui comprend une souche atténuée du virus de la varicelle et un agent stabilisant contenant du sorbitol, du mannitol, du saccharose, du dextran, un mélange d'acides aminés, de l'urée et de l'EDTA.

Dans le domaine des vaccins à virus inactivés, les compositions vaccinales prêtes à être administrées contiennent souvent des protéines d'origine animale, telles que l'albumine bovine ou humaine, la gélatine ou la caséine. Les protéines sont connues pour améliorer la conservation (stabilisation) de ces vaccins notamment les vaccins qui renferment des virus difficiles à conserver. C'est le cas des compositions vaccinales contenant du virus rabique inactivé. Frazatti-Gallina et coll. dans Vaccine (2004) vol 23, pp 511-517 souligne le rôle important des protéines dans la stabilisation du vaccin antirabique puisque l'excipient contient de l'albumine.

Néanmoins, la présence de protéines dans les vaccins, outre le fait qu'elles peuvent représenter un risque potentiel de transmission de maladies si leur origine n'est pas rigoureusement contrôlée, peuvent représenter également un risque allergique potentiel qu'on cherche à éviter, notamment lorsque les vaccins contiennent des protéines sériques telles que l'albumine ou des dérivés de l'albumine.

Il existe donc le besoin de trouver un excipient stabilisant dont la composition ne renferme pas de protéine pour stabiliser les vaccins viraux à virus entiers inactivés et en particulier pour stabiliser des vaccins contenant des virus entiers inactivés difficiles à conserver comme le virus rabique.

Il existe aussi le besoin de trouver un excipient stabilisant qui convient également pour stabiliser des vaccins à virus inactivés faiblement dosés, c'est-à-dire qui contiennent une faible quantité de protéines totales dans une dose efficace de vaccin. Ce besoin est encore plus important pour les vaccins très purifiés contenant très peu d'impuretés protéiques résiduelles. Les impuretés protéiques résiduelles même si elles représentent un risque allergique potentiel peuvent contribuer dans une certaine mesure à stabiliser les vaccins viraux inactivés faiblement dosés. Elles peuvent empêcher les phénomènes d'agrégation, de dégradation structurale ou d'adsorption du virus pouvant réduire l'efficacité du vaccin.

A cet effet, la présente invention a pour objet :
Une composition vaccinale comprenant :
a) une préparation de virus entiers inactivés et,
b) un excipient stabilisant qui comprend :
   i. une solution tampon,
   ii. un mélange d'acides aminés essentiels et non essentiels,
   iii. un disaccharide,
   iv. un polyol,
   v. un agent chélatant,
   vi. de l'urée ou un dérivé de l'urée, et
   vii. un tensioactif non ionique.

De façon préférée, le virus entier inactivé est le virus rabique.

Selon un aspect de l'invention, la composition vaccinale est également dépourvue de toute protéine sérique.

Selon un autre aspect, la composition vaccinale est également dépourvue de toute protéine exogène d'origine animale et de façon préférée dépourvue de tout produit exogène d'origine animale.

Dans encore un autre aspect, les protéines du virus représentent au moins 70% des protéines totales qui sont présentes dans la composition vaccinale.

Selon encore un autre aspect, la concentration en protéines totales dans la composition vaccinale est ≤100 µg/ml et de façon préférée ≤80 µg/ml.

Dans un aspect particulier, la quantité de protéines totales qui est contenue dans une dose efficace de la composition vaccinale est ≤100 µg.

Dans un autre aspect particulier, la quantité de protéines totales qui est contenue dans une dose efficace de la composition vaccinale est comprise entre 1 et 50µg.

Dans un autre aspect de l'invention, l'excipient stabilisant est dépourvu de toute protéine, ou de toute protéine et de tout peptide ou de façon préférée dépourvu de toute protéine, de tout peptide et de tout oligopeptide.

Selon un autre aspect, le mélange d'acides aminés essentiels et non essentiels contenu dans la composition vaccinale comprend au moins de l'arginine ou un sel d'arginine et de l'acide glutamique ou un sel d'acide glutamique.

Selon un aspect particulier, la quantité d'acides aminés essentiels et non essentiels contenue dans une dose efficace de la composition vaccinale est comprise entre 0,5 et 2,5 mg.

Selon un autre aspect, le disaccharide qui est contenu dans l'excipient est le maltose.

Selon encore un autre aspect, le polyol qui est contenu dans la composition vaccinale est le sorbitol.

Selon un aspect particulier, la quantité totale de disaccharide et de polyol contenue dans une dose efficace de la composition vaccinale est comprise entre 10 et 50 mg.

Dans un autre aspect l'agent chélatant qui est contenu dans la composition vaccinale est l'EDTA ou un sel d'EDTA.

Selon un aspect particulier, la quantité d'agent chélatant contenue dans une dose efficace de la composition vaccinale est comprise entre 0,01 et 0,1 mg.

Selon un autre aspect particulier, la quantité d'urée ou de dérivé de l'urée contenue dans une dose efficace de la composition vaccinale est comprise entre 0,3 et 1,5 mg.

Dans un autre aspect, le tensioactif non ionique qui est contenu dans la composition vaccinale est un poloxamère.

De façon préférée, le poloxamère est le poloxamère 188.

Selon un aspect particulier, la quantité de tensioactif non ionique contenue dans une dose efficace de la composition vaccinale est comprise entre 0,001 et 0,5 mg.

Selon un autre aspect, le pH de la composition vaccinale est compris entre 7,0 et 10,0 et de préférence entre 7,0 et 9,0.

Dans un autre aspect, la solution tampon qui est contenue dans la composition vaccinale est un tampon phosphate, un tampon Tris, un tampon HEPES ou un mélange de ceux-ci.

Dans un aspect particulier, la solution tampon est un tampon phosphate dont la molarité est comprise entre 10 et 100 mM.

L'invention a également pour objet un procédé de préparation d'une composition vaccinale contenant une préparation de virus entiers purifiés et inactivés selon lequel :
a. on produit une préparation de virus entiers en récoltant le surnageant d'une culture de cellules infectées par du virus,
b. on purifie et on inactive la préparation de virus entiers ou alternativement on inactive et on purifie la préparation de virus entiers, et
c. on dilue la préparation de virus entiers purifiés et inactivés dans un excipient stabilisant dont la composition comprend :
   i. une solution tampon,
   ii. un mélange d'acides aminés essentiels et non essentiels,
   iii. un disaccharide,
   iv. un polyol,
   v. un agent chélatant,
   vi. de l'urée ou un dérivé de l'urée, et
   vii. un tensioactif non ionique.

Selon un mode préféré, le procédé selon l'invention est réalisé sans introduire de produit exogène d'origine animale.

Dans un mode préféré de réalisation du procédé selon l'invention, le virus est le virus rabique.

Dans un autre aspect, le procédé selon l'invention comprend après avoir dilué la préparation de virus entiers purifiés et inactivés, une étape de répartition de la composition vaccinale ainsi obtenue dans des dispositifs de conditionnement et optionnellement une étape de lyophilisation de la composition vaccinale.

L'invention a également pour objet une composition vaccinale contenant une préparation de virus entiers purifiés et inactivés sous une forme lyophilisée obtenu selon un des modes de réalisation du procédé de l'invention.

L'invention a également pour objet un excipient stabilisant pour un vaccin à virus entiers inactivés dont la composition comprend :
i. une solution tampon,
ii. un mélange d'acides aminés essentiels et non essentiels,
iii. un disaccharide,
iv. un polyol,
v. un agent chélatant,
vi. de l'urée ou un dérivé de l'urée, et
vii. un tensioactif non ionique.

De façon préférée, la composition de l'excipient stabilisant est également dépourvue de toute protéine, ou de toute protéine et de tout peptide, ou de façon préférée dépourvu de toute protéine, de tout peptide et de tout oligopeptide.

De façon particulièrement préférée, la composition de l'excipient stabilisant est également dépourvue de tout produit d'origine animale.

### Description détaillée de l'invention

La composition vaccinale selon l'invention comprend du virus entier inactivé (ou une préparation de virus entiers inactivés) et un excipient stabilisant dont la composition comprend un tampon, un mélange d'acides aminés essentiels et non essentiels, un disaccharide, un polyol, un agent chélatant, de l'urée ou un dérivé de l'urée et un tensioactif non ionique. La composition de l'excipient remplace avantageusement les excipients de l'art antérieur qui contiennent dans leur composition une protéine. De façon particulièrement intéressante, elle stabilise les compositions vaccinales difficiles à conserver et tout particulièrement les compositions vaccinales qui contiennent comme virus entier inactivé du virus rabique, sans qu'il soit nécessaire d'ajouter une protéine.

La composition vaccinale selon l'invention est stable à la fois sous forme liquide, sous forme liquide congelée ou sous forme de lyophilisat ce qui offre une très grande souplesse d'utilisation.

L'excipient stabilisant selon l'invention préserve l'activité biologique de la composition vaccinale au cours du temps qu'elle soit sous forme congelée, liquide ou lyophilisée. La température de conservation des compositions vaccinales congelées est généralement ≤-35°C ; elle est comprise entre +2°C à +8°C pour les compositions sous forme liquide et à une température d'environ +5°C pour les compositions sous forme lyophilisée. Avantageusement, il préserve également l'activité biologique et l'intégrité physique des virus dans des compositions vaccinales conservées dans des conditions défavorables, comme la conservation à +37°C des compositions vaccinales sous forme liquide, ou lorsqu'on soumet les compositions vaccinales à une succession de décongélations et de recongélations.

L'activité biologique du virus dans la composition vaccinale est évaluée en mesurant au cours du temps la quantité d'un immunogène constitutif du virus, qui est essentiel pour induire une immunité protectrice à l'encontre du virus et/ou en testant son efficacité dans un test d'épreuve dans un modèle animal officiellement reconnu. Dans le cas d'une composition vaccinale contenant du virus rabique inactivé, la stabilité de la composition vaccinale est évaluée en mesurant au cours du temps (ou au cours de décongélations successives) le titre en virus qui est estimé sur la base de la mesure de la concentration en glycoprotéine G sous une forme non dénaturée et/ou en testant l'efficacité de la composition vaccinale au moyen du test officiel NIH. Pour évaluer le taux de glycoprotéine G, on peut utiliser une méthode ELISA, de type sandwich, qui reconnait au moins un, de façon préférée deux épitopes conformationnels de la glycoprotéine G comme cela est décrit dans l'exemple 1. Lorsqu'on met en œuvre un ELISA qui reconnait deux épitopes conformationnels de la protéine G, on utilise habituellement comme anticorps de capture, un anticorps neutralisant le virus rabique qui reconnait un épitope conformationnel localisé sur le site antigénique II de la glycoprotéine G (Journal of Clinical investigation (1989), vol. 84, p 971 à 975) et comme anticorps de révélation un anticorps neutralisant qui reconnait un épitope conformationnel localisé sur le site antigénique III de la protéine G (Biologicals (2003), vol.31 , p9 à 16). Les résultats sont exprimés en unités internationales car on utilise habituellement comme référence un étalon qui a été calibré vis-à-vis de la référence internationale du NIBSC. On peut aussi mettre en œuvre la technologie BIAcore, considérée comme alternative intéressante à la méthode ELISA, qui repose sur l'utilisation de la résonnance plasmonique de surface pour quantifier le titre en virus.

L'excipient stabilisant selon l'invention agit également en préservant l'intégrité physique des particules virales. Il empêche notamment la formation d'agrégats et/ou la dégradation de la structure des particules virales au cours du temps. Ceci peut être contrôlé au moyen d'un appareil tel que le zetasizer Nano ZS (Malvern instrument) qui détecte les agrégats et établit le profil de distribution de la taille des particules virales dans la composition vaccinale.

Le virus ou la préparation de virus est sous la forme de particules virales entières qui ont été inactivées, généralement par traitement chimique au formol, au formaldehyde ou à la β propiolactone. D'autres procédés d'inactivation comme celui qui est décrit dans WO 2005/093049 peuvent également être mis en œuvre. Les préparations de virus entiers inactivés principalement utilisées contiennent des virus enveloppés car elles sont souvent plus difficiles à conserver et requièrent l'utilisation de compositions d'excipients spécifiques pour assurer leur conservation. Il s'agit notamment de préparations de virus entiers inactivés de l'encéphalite japonaise. De façon particulièrement préférée, les préparations de virus entiers inactivés contiennent du virus rabique.

Les préparations de virus proviennent de récoltes qui sont généralement sous la forme de prélèvements de surnageants de culture de cellules infectées par le virus. On peut utiliser des milieux classiques contenant du sérum d'origine animale pour produire le stock de cellules et infecter les cellules. Avantageusement, les milieux qui servent à la culture et à l'infection des cellules ne contiennent pas de protéine sérique, ni même de produit d'origine animale. Les protéines qui sont éventuellement présentes dans ces milieux sont généralement des protéines de bas poids moléculaire (≤ 10 KD) ou plutôt des peptides à des concentrations très faibles ce qui diminue d'autant les risques allergiques. C'est le cas par exemple des milieux commercialisés sous les dénominations VP SFM (InVitrogen), Opti Pro ™ serum-free (InVitrogen), Episerf (InVitrogen), Ex-cell ® MDCK (Sigma-Aldrich), Ex-Cell ™ Vero (SAFC biosciences) MP-BHK® serum free (MP Biomedicals), SFC-10 BHK express serum free (Promo cell), SFC-20 BHK express protein free (Promo cell), HyQ PF Vero (Hyclone Réf. SH30352.02), Hyclone SFM4 Megavir, le milieu MDSS2 (Axcell biotechnology), le milieu DMEM Iscove modifié (Hyclone), les milieux nutritifs de Ham (Ham-F10, Ham-F12), le milieu de leibovitz L-15 (Hyclone). Par exemple, les récoltes de virus rabiques sont obtenues à partir d'un stock de cellules Vero qui ont été produites puis infectées en utilisant des milieux de culture et d'infection virale qui sont de préférence dépourvus de toute protéine sérique, de toute protéine d'origine animale et même de tout produit d'origine animale, tel que le milieu VP SFM.

Avantageusement la préparation de virus entiers et inactivés contenue dans la composition vaccinale selon l'invention est très purifiée. Habituellement, on purifie puis on inactive ou inversement on inactive puis on purifie le virus qui provient des récoltes. Lorsque toutes les étapes de production et de purification du virus sont réalisées sans utiliser de protéine sérique, sans utiliser de protéine exogène d'origine animale, ou même sans utiliser de produit exogène d'origine animale, avantageusement la composition vaccinale selon l'invention est dépourvue de toute protéine sérique pour réduire au maximum les risques allergiques, et dépourvue de toute protéine exogène d'origine animale ou même de tout produit d'origine animale pour réduire au maximum les risques de transmission de maladies. Par «protéine ou produit d'origine animale» on entend une protéine ou un produit dont le procédé de fabrication comprend au moins une étape où on utilise une matière provenant de l'animal ou de l'homme. Par «protéine ou produit exogène» on entend une protéine ou un produit qui est introduit dans l'une des étapes du procédé de production et/ou de purification du virus. Par exemple, les protéines ou les produits qui sont contenus éventuellement dans la composition des milieux de culture, les enzymes tels que la trypsine ou la benzonase qui sont utilisées lors des étapes de production et/ou de purification du virus sont des protéines ou des produits exogènes. Les protéines ou les produits exogènes sont d'origine animale dès lors que leur procédé de fabrication comprend au moins une étape où on utilise une matière provenant de l'animal ou de l'homme. Les protéines ou les produits exogènes sont d'origine non animale lorsqu'ils sont fabriqués par d'autres moyens, par exemple en utilisant une matière végétale, par synthèse chimique ou par recombinaison génétique en utilisant des levures, des bactéries ou des plantes. Les protéines ou les produits provenant des cellules à partir desquelles les virus sont produits pour obtenir la composition vaccinale selon l'invention sont par contre des protéines ou des produits endogènes car ils sont produits (ou relargués) en même temps que les virus.

Dans le cadre de la présente invention on peut mettre en œuvre avec profit des procédés de purification particulièrement performants qui conduisent à l'obtention de préparations virales très pures. On cite à titre d'exemple le procédé de purification de virus qui comprend une chromatographie échangeuse d'anions suivie d'une chromatographie échangeuse de cations et complété par une chromatographie d'affinité par chélation métallique qui est décrit dans WO 97/06243. Pour purifier et inactiver le virus rabique à partir d'un surnageant de culture de cellules infectées, une méthode de choix consiste à utiliser le procédé qui est décrit dans la demande de brevet déposé en France le 14 avril 2009 sous le numéro 0952310 qui comprend une étape de chromatographie par échange de cations sur un support comprenant une matrice de polyméthacrylate sur laquelle sont greffés des groupemements sulfoisobutyls, une étape de traitement à la benzonase, une étape d'ultracentrifugation sur gradient de saccharose et une étape d'inactivation avec la β propiolactone. Les préparations de virus rabiques entiers et inactivés obtenues sont particulièrement pures, mais sont plus difficiles à conserver (stabiliser) car il y a très peu d'impuretés protéiques résiduelles. Cette difficulté est d'autant plus grande qu'il y a de petites quantités de virus dans la préparation vaccinale

Grâce à la composition de l'excipient, la composition vaccinale selon l'invention reste stable même dans les cas où la concentration en protéines totales est ≤100 µg/ml, ≤80 µg/ml, ≤50 µg/ml ou même ≤20 µg/ml. Généralement les protéines du virus représentent au moins 70% des protéines totales, de façon préférée au moins 80% des protéines totales et de façon particulièrement préférée au moins 90% des protéines totales qui sont présentes dans la composition vaccinale selon l'invention. Dans une dose efficace de vaccin cela représente habituellement une quantité en protéines totales ≤ 100 µg ou ≤ 50 µg ou même ≤ 20 µg. A titre indicatif, la quantité d'ADN résiduelle est quant à elle <100 pg, et de façon préférée <50 pg par dose efficace de vaccin. Par «dose efficace de vaccin (ou dose efficace d'une composition vaccinale)» on entend la quantité de virus inactivés contenue dans une dose qui est nécessaire pour induire une immunité protectrice chez l'homme ou l'animal après administration selon la voie d'immunisation et le protocole d'immunisation qui sont préconisés dans le cadre d'une primo vaccination ou d'une vaccination de rappel. Dans le cas de la vaccination contre le virus rabique, l'efficacité du vaccin antirabique est déterminé au moyen du test officiel admis par l'OMS, le test NIH (décrit dans la monographie rage de l'OMS (WHO Technical Series Report 941- janvier 2007). Une dose de vaccin antirabique inactivé est efficace lorsqu'elle contient au moins 2,5 UI selon ce test. L'administration de cette dose par voie intramusculaire chez l'homme selon les protocoles de vaccination ou de séro-vaccination habituellement préconisés induit le développement d'une immunité protectrice contre la rage.

Les «protéines totales» correspondent à toutes les protéines qui sont présentes dans la composition vaccinale. Elles sont représentées par les protéines virales, et les protéines résiduelles qui n'ont pas été éliminées lors de la purification comme les protéines cellulaires, les protéines des milieux de culture cellulaire et d'infection virale, et les protéines qui ont été éventuellement introduites dans le procédé de purification (par exemple la benzonase dans le cas du procédé de purification du virus rabique tel que mentionné ci-dessus). On utilise généralement la méthode de Bradford pour quantifier les protéines totales. Pour quantifier la proportion de protéines virales parmi les protéines totales on réalise sur un échantillon de la composition vaccinale une électrophorèse sur gel de polyacrylamide en conditions dénaturante et réductrice suivie d'une révélation au bleu de coomassie et d'une analyse densitométrique du profil électrophorétique Dans le cas d'une composition vaccinale contenant du virus rabique entier inactivé, les protéines virales représentées par la glycoprotéine d'enveloppe G, La nucléoprotéine N, la phosphoprotéine P, la protéine matricielle M et l'ARN polymerase ARN dépendante L sont facilement identifiables sur une électrophorèse en gel de polycrylamide.. La quantité de protéines totales contenue dans une dose efficace de la composition vaccinale selon l'invention est généralement comprise entre 1µg et 50µg, de façon préférée entre 2µg et 20µg. De façon particulièrement préférée au moins 70%, au moins 75%, au moins 80%, au moins 85% ou même au moins 90% des protéines totales sont des protéines virales. L'excipient selon l'invention est particulièrement avantageux puisqu'il stabilise des compositions vaccinales difficiles à conserver, telles que des compositions vaccinales contenant du virus rabique entier inactivé, et dans lesquelles on retrouve dans une dose vaccinale efficace :
- une quantité de protéines virales qui représente au moins 70%, des protéines totales et/ou
- moins de 100µg de protéines totales, le plus souvent une quantité de protéines totales comprise entre 1µg et 50µg, de façon préférée entre 2µg 20µg.

La composition de l'excipient selon l'invention est de façon préférée dépourvue de toute protéine, de tout peptide et même de tout oligopeptide et généralement ne contient pas de produit d'origine animale pour diminuer au maximum les problèmes de sécurité biologique et/ou allergiques qui peuvent être associés à leur utilisation.

Dans le contexte de la présente invention, l'expression « l'excipient stabilisant est dépourvu de toute protéine » doit être comprise comme signifiant un excipient stabilisant dont la composition est dépourvue de toute macromolécule biologique comprenant une chaîne de plus de 50 acides aminés liés entre eux au moyen de liaisons peptidiques. L'expression « l'excipient stabilisant est dépourvu de toute protéine et de tout peptide » doit être comprise comme signifiant un excipient stabilisant dont la composition est dépourvue de toute macromolécule biologique comprenant une chaîne de plus de 20 acides aminés liés entre eux au moyen de liaisons peptidiques. L'expression « l'excipient stabilisant est dépourvu de toute protéine, de tout peptide et de tout oligopeptide » doit être comprise comme signifiant un excipient stabilisant dont la composition est dépourvue de toute molécule biologique comprenant des acides aminés liés entre eux par une ou plusieurs liaisons peptidiques. Par exemple un dipeptide qui contient deux acides aminés liés entre eux par une seule liaison peptidique est exclut de la composition d'un l'excipient stabilisant dépourvu de toute protéine, de tout peptide et de tout oligopeptide mais peut faire partie de la composition d'un 'excipient stabilisant dépourvu de toute protéine et de tout peptide.

Le mélange d'acides aminés qui fait partie de la composition de l'excipient comprend au moins un acide aminé essentiel dans l'ensemble des acides aminés essentiels représenté par la cystine, la tyrosine, l'arginine, l'histidine, l'isoleucine, la leucine, la lysine, la méthionine, la phénylalanine, la thréonine, le tryptophane et la valine et au moins un acide aminé non essentiel dans l'ensemble des acides aminés non essentiels représenté par l'acide aspartique, l'acide glutamique, l'alanine, l'asparagine, la glutamine, la glycine, la proline et la sérine. Les acides aminés à fonction acide peuvent être sous la forme d'acides ou de sels. Il en est de même pour les acides aminés basiques.

De façon préférée, l'excipient selon l'invention contient au moins de l'arginine ou un sel d'arginine tel que le chlorhydrate d'arginine et de l'acide glutamique ou un sel d'acide glutamique tel que le glutamate de sodium. En définitive, le mélange d'acides aminés comprend de façon préférée de 1 à 12 acides aminés essentiels parmi lesquels on retrouve au moins l'arginine ou un sel de celui ci et de 1 à 8 acides aminés non essentiels parmi lesquels on retrouve au moins l'acide glutamique ou un sel de celui ci. La concentration totale en acides aminés dans la composition vaccinale est généralement ≤ 20g/l, le plus souvent comprise entre 2g/l et 10g/l. Dans une dose efficace de la composition vaccinale selon l'invention cela représente une quantité totale en acides aminés essentiels et non essentiels ≤ 5mg, de façon préférée à une quantité qui est comprise entre 0,5 et 2,5 mg et de façon particulièrement préférée entre 0,8 et 1,8 mg. Le ratio entre la quantité d'arginine (sous forme de chlorhydrate) et la quantité totale d'acides aminés contenue dans l'excipient est en général supérieur à 0,5 tandis que le ratio entre la quantité d'acide glutamique (sous forme glutamate de sodium) et la quantité totale d'acides aminés contenue dans l'excipient est inférieur à 0,5.

L'excipient selon l'invention contient également un disaccharide. On exclut les disaccharides qui sont obtenus à partir d'une matière première d'origine animale comme le lait. Comme disaccharide convenant à l'objet de l'invention, on cite le saccharose, le maltose ou le tréhalose, mais de façon préférée on utilise le maltose seul ou en combinaison avec un autre disaccharide comme le saccharose. Le polyol qui est également inclus dans la composition de l'excipient est d'origine non animale. Il s'agit principalement d'un hexol tel que le sorbitol et/ou le mannitol. De façon préférée on utilise le sorbitol car le mannitol a l'inconvénient de cristalliser lorsque la composition vaccinale selon l'invention est sous une forme lyophilisée. La concentration totale en disaccharide et en polyol dans la composition vaccinale est généralement comprise entre 30g/l et 100g/l. De façon préférée l'excipient selon l'invention contient du maltose et du sorbitol. Dans une dose efficace de la composition vaccinale cela correspond habituellement à une quantité comprise entre 10 et 50 mg et de façon préférée à une quantité comprise entre 20 et 25 mg. La quantité de maltose représente généralement au moins 70% de la quantité totale de maltose et de sorbitol.

L'agent chélatant est également un des composants de l'excipient selon l'invention. Il s'agit principalement de l'EDTA (Acide éthylène diamine tétra acétique) ou d'un sel de celui-ci (Na⁺, K⁺,....). La concentration en EDTA ou en sel d'EDTA dans la composition vaccinale est généralement comprise entre 0,02 et 0,5g/l et de façon préférée entre 0,02 et 0,2g/l. Dans une dose efficace de la composition vaccinale cela correspond habituellement à une quantité comprise entre 0,01 et 0,1 mg, de façon préférée entre 0,01 et 0,05 mg et de façon particulièrement préférée entre 0,01 et 0,04 mg d'EDTA ou de sel d'EDTA.

L'urée ou un dérivé de l'urée tel que l'allylurée, l'acétamide, le méthylcarbamate ou le butylcarbamate fait également partie de la composition de l'excipient. De façon préférée, la composition vaccinale contient de l'urée à une concentration généralement comprise entre 1g et 10g/l, de façon préférée à une concentration comprise entre 1g et 5g/l. Dans une dose efficace de la composition vaccinale cela correspond habituellement à une quantité comprise entre 0,3 et 3 mg, de façon préférée entre 0,3 et 1,5 mg et de façon particulièrement préférée entre 0,4 et 1,2 mg d'urée.

L'excipient selon l'invention comprend également un tensioactif non ionique qui contribue à la stabilisation de la composition vaccinale. Sans être lié par la théorie, il contribue au maintien de l'activité biologique du virus et au maintien de l'intégrité physique des particules virales en évitant la formation d'agrégats ou la dégradation de la structure des virus. Il peut également éviter l'adsorption du virus sur les parois du conditionnement, en particulier lorsque les parois sont en verre ou en plastique. Le tensioactif non ionique convenant à l'objet de l'invention est produit à partir d'une matière première d'origine non animale et est compatible sur le plan pharmaceutique avec une administration par voie parentérale du produit. Comme classe de tensioactif non ionique convenant particulièrement à l'objet de l'invention on cite les poloxamères qui sont des « copolymères en bloc » d'oxyde d'éthylène et d'oxyde de propylène ayant pour formule chimique HO(C₂H₄O)ₐ(C₃H₆O)_{b}(C₂H₄O)ₐH selon laquelle a désigne le nombre d'unités d'oxyde d'éthylène et b désigne le nombre d'unités d'oxyde de propylène. Les poloxamères ont un poids moléculaire allant généralement de 1000 à 16000 daltons. Les poloxamères ayant un intérêt particulier ont un poids moléculaire compris entre 5000 et 15500 daltons. Les poloxamères sont commercialisés notamment sous la dénomination commerciale de Pluronic® parmi lesquels le pluronic® F68, ou poloxamère 188 qui désigne un poloxamère sous forme solide à température ambiante dont le poids moléculaire de la partie polyoxypropylène est d'environ 1750 daltons et dont la partie polyoxyéthylène représente environ 80% du poids total de la molécule. Parmi les poloxamères, on recommande particulièrement le poloxamère 188 (pluronic® F68). Eventuellement, on peut utiliser un ester de sorbitan, en particulier un ester polyoxyéthyléné de sorbitan commercialisé sous la dénomination commerciale de Tween®, comme le tween® 20 (monolaurate de polyoxyethylene (20) sorbitan ou polysorbate 20) ou le tween® 80 (monooleate de polyoxyethylene (20) sorbitan ou polysorbate 80). Le tensioactif non ionique convenant à l'objet de l'invention est utilisé à une concentration très faible qui préserve la structure et la taille des particules virales inactivées qui doit rester similaire à celle des virus vivants. A trop forte concentration, le tensioactif peut dissocier ou modifier la structure des particules virales en particulier celle des virus enveloppés, ce qui peut affecter l'immunogénicité de la composition vaccinale. Lorsque le pluronic® F68 est utilisé comme tensioactif, la concentration totale dans la composition vaccinale est ≤1g/l, généralement comprise entre 0,005 g/l et 1g/l, et de façon particulièrement préférée entre 0,01g/l et 0,1g/l. Dans cette gamme de concentration, le poloxamère 188 n'exerce pas d'action adjuvante sur le système immunitaire. Dans une dose efficace de la composition vaccinale cela correspond habituellement à une quantité comprise entre 0,001 mg et 0,5 mg et de façon préférée entre 0,003 et 0,3 mg. De façon très préférée, la composition vaccinale contient du poloxamère 188 ou éventuellement un mélange de poloxamère 188 et de polysorbate 20.

Le tampon de l'excipient selon l'invention est choisi de telle sorte que le pH de la composition vaccinale soit compris entre 7,0 et 10,0 de façon préférée entre 7,0 et 9,0 et de façon particulièrement préférée entre 7,3 et 8,3. C'est dans cette gamme de pH que la thermostabilité physique des particules de virus inactivées est maximale, en particulier celle des particules de virus rabique. Les études de diagramme de phase montrent qu'il faut chauffer la préparation de virus rabiques à une température d'au moins 60°C pour observer une agrégation des particules de virus rabiques lorsque le pH est compris entre 7,3 et 8,3 alors qu'à un pH < 6,0 on observe une agrégation importante à température ambiante. La solution tampon est habituellement un tampon phosphate, un tampon Tris ou un tampon HEPES, ou un mélange de ceux-ci. Leur concentration est généralement comprise entre 10 et 100mM. De façon préférée, la composition de l'excipient comprend un tampon phosphate dont la molarité est habituellement comprise entre 10 et 100 mM, ou un tampon phosphate et un tampon Tris en mélange.

Une composition vaccinale particulièrement préférée selon l'invention comprend :
une préparation de virus rabiques entiers et inactivés, et un excipient stabilisant qui comprend :
un tampon phosphate ;
un mélange d'acides aminés essentiels et non essentiels contenant au moins de l'arginine ou un sel d'arginine et de l'acide glutamique ou un sel d'acide glutamique ;
du maltose ;
du sorbitol ;
de l'EDTA ou un sel d'EDTA;
de l'urée;
du poloxamère 188,
le pH de la composition vaccinale étant compris entre 7,3 et 8,3.

Eventuellement du tween 20 peut être incorporé en tant que tensioactif non ionique additionnel à cette composition vaccinale. Cette composition vaccinale est avantageusement dépourvue de toute protéine sérique et de tout produit exogène d'origine animale et contient généralement une préparation de virus rabiques très purifiés. La concentration en poloxamère 188 dans la composition vaccinale est <1g/l et de façon très préférée entre 0,01 g/l et 0,1g/l. La molarité du tampon phosphate est généralement comprise entre 10 et 100 mM. L'arginine et l'acide glutamique ou leurs sels respectifs sont les composants très majoritaires du mélange d'acides aminés car ils représentent généralement en poids au moins les 2/3 de la quantité totale d'acides aminés essentiels et non essentiels. Le mélange d'acides aminés essentiels et non essentiels est habituellement à une concentration comprise entre 2 et 10 g/l, la concentration totale en maltose et sorbitol est habituellement comprise entre 50 et 100 g/l, la concentration en EDTA ou en sel d'EDTA est habituellement comprise entre 0,02 et 0,2 g/l et l'urée est à une concentration habituellement comprise entre 1 et 5 g/l. La concentration en protéines totales dans la composition vaccinale est habituellement entre 5 et 50 µg/ml. Dans une dose efficace de vaccin anti rabique, cela représente habituellement une quantité d'acides aminés essentiels et non essentiels comprise entre 0,5 mg et 2,5 mg, une quantité de maltose et de sorbitol comprise entre 10 et 50 mg, une quantité d'EDTA ou de sel d'EDTA comprise entre 0,01 mg et 0,1 mg, une quantité d'urée comprise entre 0,3 et 1,5 mg, une quantité de poloxamère 188 comprise entre 0,001 et 0,5 mg et une quantité de protéines totales compris entre 2 et 20 µg.

Le procédé de préparation d'une composition vaccinale selon l'invention outre les étapes de production, de purification et d'inactivation de la préparation de virus comprend une étape où l'on introduit l'excipient stabilisant dans la préparation de virus entiers purifiés et inactivés et une étape de répartition de la composition ainsi obtenue dans des dispositifs de conditionnement. En général, on dilue la préparation de virus dans l'excipient stabilisant selon l'invention avant de répartir la composition vaccinale dans les dispositifs de conditionnement.

La composition vaccinale selon l'invention peut se présenter sous la forme de vrac : dans ce cas, la concentration en virus entiers inactivés est plus élevée que celle qui existe dans la composition vaccinale une fois répartie, mais la composition de l'excipient est la même. Le vrac est généralement conservé congelé à une température <-35°C. L'étape de répartition de la composition vaccinale se fait alors en décongelant le vrac que l'on dilue ensuite dans l'excipient stabilisant de façon à obtenir la concentration virale souhaitée. On obtient alors le produit final vrac qui est ensuite réparti dans les dispositifs de conditionnement. Pour garantir la stérilité de la composition vaccinale on peut également incorporer une étape de filtration stérilisante, par exemple sur membrane filtrante de 0,2 µm, avant l'étape de répartition.

Le vrac est généralement obtenu en diafiltrant la préparation de virus purifiés et inactivés obtenue à la fin de la mise en œuvre du procédé de purification dans une solution tampon qui correspond en général au tampon de l'excipient. Si par exemple le tampon de l'excipient est un tampon phosphate 50 mM, on utilise un tampon phosphate 50 mM comme tampon de diafiltration. Si l'on souhaite augmenter la concentration en virus l'étape de diafiltration est précédée d'une étape d'ultrafiltration. On utilise une membrane ultrafiltrante ayant un seuil de coupure bas, compris généralement entre 5 kDa et 100 kDa, de façon préférée entre 8 kDa et 50 kDa, et de façon particulièrement préférée autour de 10 kDa de façon à retenir au maximum le virus dans le rétentat et éliminer les sels qui peuvent avoir un impact négatif sur le procédé de lyophilisation. On mélange ensuite le rétentat contenant la préparation de virus avec l'excipient stabilisant pour obtenir un vrac dont la composition correspond à la composition vaccinale selon l'invention. On peut également utiliser comme tampon de diafiltration un tampon qui a la même composition que l'excipient stabilisant pour obtenir en une seule étape le vrac.

Le procédé de préparation d'une composition vaccinale selon l'invention contenant du virus rabique entier et inactivé comprend les étapes suivantes : on produit une préparation de virus entiers en récoltant le surnageant d'une culture de cellules Vero infectées par du virus, en utilisant des milieux de culture cellulaire et d'infection virale qui de préférence sont dépourvus de toute protéine sérique et de toute produit d'origine animale, comme par exemple en utilisant le milieu VP SFM. On purifie et on inactive le virus en utilisant notamment le procédé tel que décrit dans la demande de brevet déposé en France le 14 avril 2009 sous le numéro 0952310 qui comprend une étape de chromatographie par échange de cations sur un support comprenant de façon préférée une matrice à base de polymétacrylate sur laquelle sont greffés des groupements sulfo isobutyl, un traitement avec une benzonase recombinante, une étape d'ultracentrifugation sur gradient de saccharose et une étape d'inactivation avec la β propiolactone. La préparation de virus rabiques entiers et inactivés obtenue est très pure, et est dépourvue de toute protéine sérique et de tout produit exogène d'origine animale ; la teneur résiduelle en ADN est <100pg/ml et les protéines virales représentent au moins 70% des protéines totales. On prépare ensuite un vrac selon les modalités décrites dans le paragraphe précédent que l'on conserve en général à une température <-35°C. On dilue ensuite le vrac à la concentration virale souhaitée dans l'excipient selon l'invention avant de le répartir dans les dispositifs de conditionnement. On introduit habituellement entre 0,1 ml et 1 ml de la composition vaccinale dans chaque dispositif de conditionnement de telle sorte que la composition une fois répartie contienne au moins une dose efficace de vaccin. Les dispositifs de conditionnement servant à la répartition du produit final vrac sont habituellement sous la forme de flacons (en verre ou en plastique) ou de seringues mais tout autre dispositif de conditionnement compatible avec la pratique de la vaccination peut être également utilisé.

L'analyse granulométrique des compositions vaccinales selon l'invention contenant du virus rabique purifié au moyen de l'appareil zétasizer Nano ZS (Malvern Instrument) qui mesure le mouvement brownien des particules sur la base de la diffusion «quasi élastique» de la lumière (Dynamic Light scattering) montre l'existence d'une seule population homogène de particules virales comprise entre 100 et 300 nm avec une valeur moyenne autour de 180 nm qui correspond à la taille moyenne du virus rabique sauvage. On ne détecte pas d'agrégats ni de modifications du profil de distribution de la taille des particules virales au cours de la conservation des compositions vaccinales selon l'invention.

Les compositions vaccinales sont stables sous forme liquide pendant au moins 3 mois à une température de 2-8°C et pendant au moins 1 mois à 23-27°C (voir exemple 3). Elles peuvent être également conservées pendant au moins 12 mois et de façon préférée pendant au moins 24 mois sous forme congelée à une température ≤-35°C. Les compositions vaccinales peuvent être également conservées sous forme lyophilisée en utilisant un procédé classique de lyophilisation. La composition de l'excipient selon l'invention n'entraine pas de perte significative de virus durant l'étape de lyophilisation. Une méthode de lyophilisation consiste à congeler la composition à une température inférieure à -40°C puis à dessécher le produit en deux temps, la dessiccation primaire se faisant à une température voisine de -15°C sous 80 µbar et la dessiccation secondaire se faisant à une température voisine de + 40°C sous 80 µbar. Les doses de vaccin lyophilisées ont un taux résiduel d'humidité ≤3% et sont stables à une température de 2-8°C pendant au moins 18 mois (voir exemple 3). En définitive, la composition vaccinale antirabique telle que décrite dans l'invention s'avère aussi stable qu'une composition vaccinale de l'art antérieur commercialisée sous la dénomination de VeroraB™ qui renferme dans une dose de vaccin une quantité de protéines totales qui est au moins 100 fois plus importante que celle que l'on peut rencontrer dans une dose efficace de cette composition vaccinale qui est généralement ≤50 µg. Par ailleurs, la composition de l'excipient selon l'invention, permet avantageusement de conserver durablement des compositions vaccinales sous forme liquide ou congelée même si la forme préférée de conservation est la forme lyophilisée.

La composition vaccinale est administrable directement au sujet à vacciner lorsqu'elle est conservée sous forme liquide ou après décongélation si elle est conservée congelée. Lorsqu'elle est sous une forme lyophilisée, le lyophilisat est repris extemporanément dans un diluent qui est habituellement une solution saline, comme par exemple une solution de chlorure de sodium hypotonique. Le diluent peut contenir en outre un tensioactif à très faible concentration dont la structure chimique est compatible au plan pharmaceutique avec une utilisation par voie parentérale. Il s'agit habituellement du tween® 20, du tween® 80, ou du pluronic® F68 que l'on utilise à une concentration trop faible pour exercer une action adjuvante. La concentration du surfactant dans le diluent est généralement ≤0,1% (p/p).

Lorsque le vaccin est sous une forme lyophilisée il se présente habituellement sous la forme d'un kit avec deux conditionnements, le premier (généralement sous la forme d'un flacon) contient la composition vaccinale lyophilisée, le second (généralement sous la forme d'un flacon ou d'une seringue) contient le diluent. On peut également utiliser une seringue de type «by pass» dans laquelle la composition vaccinale se trouve dans la partie inférieure de la seringue tandis que la partie supérieure contient le diluent.

L'invention a finalement pour objet un excipient stabilisant pour un vaccin à virus entiers inactivés, en particulier pour un vaccin à virus rabiques entiers inactivés qui comprend :
a. une solution tampon,
b. un mélange d'acides aminés essentiels et non essentiels,
c. un disaccharide,
d. un polyol,
e. un agent chélatant,
f. de l'urée ou un dérivé de l'urée, et
g. un tensioactif non ionique.

De façon préférée, l'excipient est dépourvu de toute protéine, de façon préféré de tout peptide et de toute protéine et de façon encore plus préférée de toute protéine, de tout peptide et de tout oligopeptide. Il est également de façon très préféré dépourvu de tout produit d'origine animale.

La Présente invention concerne en particulier les différents aspects suivants :
1. Une composition vaccinale comprenant :
   a) Une préparation de virus entiers inactivés et,
   b) un excipient stabilisant qui comprend :
      i. une solution tampon,
      ii. un mélange d'acides aminés essentiels et non essentiels,
      iii. un disaccharide,
      iv. un polyol,
      v. un agent chélatant,
      vi. de l'urée ou un dérivé de l'urée, et
      vii. un tensioactif non ionique.
2. Une composition selon l'aspect 1, selon laquelle le virus entier inactivé est le virus rabique.
3. Une composition selon l'aspect 1 ou 2, caractérisée en ce qu'elle est également dépourvue de toute protéine sérique.
4. Une composition selon l'un des aspects 1 à 3, caractérisée en ce qu'elle est également dépourvue de toute protéine exogène d'origine animale et de façon préférée dépourvue de tout produit exogène d'origine animale.
5. Une composition selon l'un des aspects 1 à 4, caractérisée en ce que les protéines du virus représentent au moins 70% des protéines totales présentes dans la composition vaccinale.
6. Une composition selon l'un des aspects 1 à 5, caractérisée en ce que la concentration en protéines totales est ≤100 µg/ml et de façon préférée ≤80 µg/ml.
7. Une composition selon l'un des aspects 1 à 6, caractérisée en ce que la quantité de protéines totales qui est contenue dans une dose efficace de la composition vaccinale est ≤100 µg.
8. Une composition selon l'un des aspects 1 à 7, caractérisée en ce que la quantité de protéines totales qui est contenue dans une dose efficace de la composition vaccinale est comprise entre 1 et 50 µg.
9. Une composition selon l'un des aspects 1 à 8, caractérisée en ce que l'excipient stabilisant est dépourvu de toute protéine, ou de toute protéine et de tout peptide, ou de façon préférée dépourvu de toute protéine, de tout peptide et de tout oligopeptide.
10. Une composition selon l'un des aspects 1 à 9, caractérisée en ce que le mélange d'acides aminés essentiels et non essentiels comprend au moins de l'arginine ou un sel d'arginine et de l'acide glutamique ou un sel d'acide glutamique.
11. Une composition selon l'un des aspects 1 à 10, caractérisée en ce que la quantité d'acides aminés essentiels et non essentiels contenue dans une dose efficace de la composition vaccinale est comprise entre 0,5 et 2,5 mg.
12. Une composition selon l'un des aspects 1 à 11, caractérisée en ce que le disaccharide est le maltose.
13. Une composition selon l'un des aspects 1 à 12, caractérisée en ce que le polyol est le sorbitol.
14. Une composition selon l'un des aspects 1 à 13, caractérisée en ce que la quantité totale de disaccharide et de polyol contenue dans une dose efficace de la composition vaccinale est comprise entre 10 et 50 mg.
15. Une composition selon l'un des aspects 1 à 14, caractérisée en ce que l'agent chélatant est l'EDTA ou un sel d'EDTA.
16. Une composition selon l'un des aspects 1 à 15, caractérisée en ce que la quantité d'agent chélatant contenue dans une dose efficace de la composition vaccinale est comprise entre 0,01 et 0,1 mg.
17. Une composition selon l'un des aspects 1 à 16, caractérisée en ce que la quantité d'urée ou de dérivé de l'urée contenue dans une dose efficace de la composition vaccinale est comprise entre 0,3 et 1,5 mg.
18. Une composition selon l'un des aspects 1 à 17, caractérisée en ce que le tensioactif non ionique est un poloxamère.
19. Une composition selon l'un des aspects 1 à 18, caractérisée en ce que le tensioactif non ionique est le poloxamère 188.
20. Une composition selon l'un des aspects 1 à 19, caractérisée en ce que la quantité de tensioactif non ionique contenue dans une dose efficace de la composition vaccinale est comprise entre 0,001 et 0,5 mg.
21. Une composition selon l'un des aspects 1 à 20, caractérisée en ce que le pH de la composition vaccinale est compris entre 7,0 et 10,0 et de préférence entre 7,0 et 9,0.
22. Une composition selon l'un des aspects 1 à 21, caractérisée en ce que la solution tampon est un tampon phosphate, un tampon Tris, un tampon HEPES ou un mélange de ceux-ci.
23. Une composition selon l'un des aspects 1 à 22, caractérisée en ce que la solution tampon est un tampon phosphate dont la molarité est comprise entre 10 et 100 mM.
24. Un procédé de préparation d'une composition vaccinale contenant une préparation de virus entiers purifiés et inactivés, selon lequel:
   a. on produit une préparation de virus entiers en récoltant le surnageant d'une culture de cellules infectées par du virus,
   b. on purifie et on inactive la préparation de virus entiers ou alternativement on inactive puis on purifie la préparation de virus entiers, et
   c. on dilue la préparation de virus entiers purifiés et inactivés dans un excipient stabilisant dont la composition comprend :
      i. une solution tampon,
      ii. un mélange d'acides aminés essentiels et non essentiels,
      iii. un disaccharide,
      iv. un polyol,
      v. un agent chélatant,
      vi. de l'urée ou un dérivé de l'urée, et
      vii. un tensioactif non ionique.
25. Un procédé selon l'aspect 24, caractérisé en ce qu'il est réalisé sans introduire de produit exogène d'origine animale.
26. Un procédé selon l'aspect 24 ou 25, selon lequel le virus est le virus rabique.
27. Un procédé selon l'un des aspects 24 à 26, selon lequel après avoir dilué la préparation de virus entiers purifiés et inactivés, on répartit la composition vaccinale ainsi obtenue dans des dispositifs de conditionnement, et optionnellement on lyophilise la composition vaccinale.
28. Une composition vaccinale contenant une préparation de virus entiers purifiés et inactivés sous une forme lyophilisée obtenue selon le procédé de l'aspect 27.
29. Un excipient stabilisant pour un vaccin à virus entiers inactivés dont la composition comprend :
   i. une solution tampon,
   ii. un mélange d'acides aminés essentiels et non essentiels,
   iii. un disaccharide,
   iv. un polyol,
   v. un agent chélatant,
   vi. de l'urée ou un dérivé de l'urée, et
   vii. un tensioactif non ionique.
30. Un excipient stabilisant selon l'aspect 29, caractérisé en ce qu'il est également dépourvu de toute protéine, ou de toute protéine et de tout peptide, ou de façon préférée dépourvu de toute protéine, de tout peptide et de tout oligopeptide
31. Un excipient selon l'easpect 30, caractérisé en ce qu'il est également dépourvu de tout produit d'origine animale.

La présente invention sera mieux comprise à la lumière des exemples suivants qui servent à illustrer l'invention sans pour autant limiter le contenu.

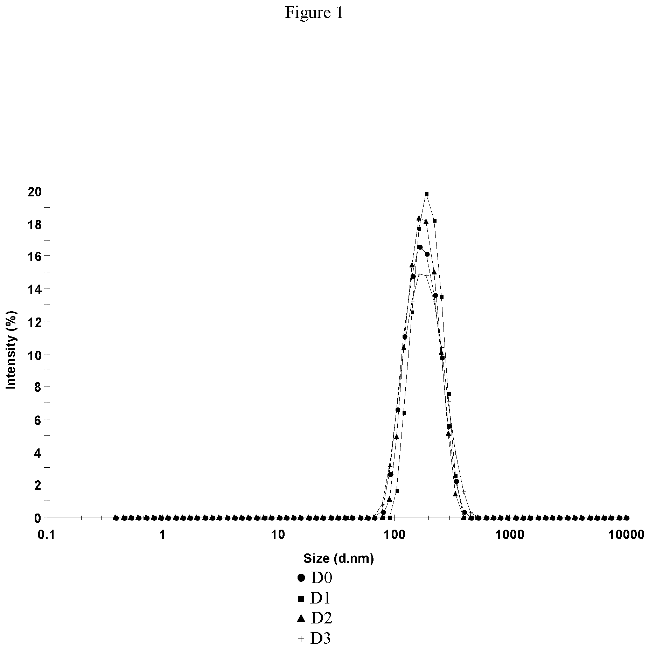

La figure 1 est une superposition des profils de distribution de la taille des particules virales obtenus par « dynamic light scattering (DLS) » au cours d'un cycle de 3 décongélations et recongélations successives d'une composition vaccinale antirabique dans laquelle la composition de l'excipient stabilisant est F04 + 0,001% de poloxamère 188. ● D0 (avant congélation),■ D1 (après la première décongélation); ▲ D2 (après la seconde décongélation) et + D3 (après la troisième décongélation).

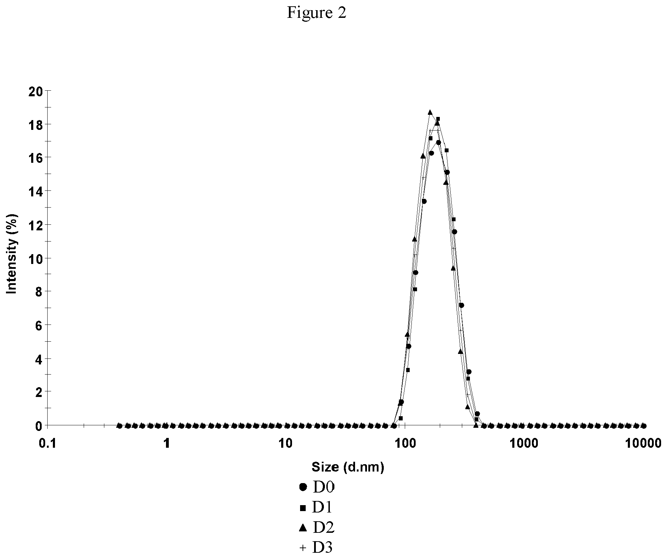

La figure 2 est une superposition des profils de distribution de la taille des particules virales obtenus par « dynamic light scattering (DLS) » au cours d'un cycle de 3 décongélations et recongélations successives d'une composition vaccinale antirabique dans laquelle la composition de l'excipient stabilisant est F04 + 0,01% de poloxamère 188. • D0 (avant congélation),■ D1 (après la première décongélation); ▲ D2 (après la seconde décongélation) et + D3 (après la troisième décongélation).

### Exemple 1 : Influence de l'excipient sur la stabilité d'une composition vaccinale contenant du virus rabique purifié et inactivé sous la forme de vrac.

### 1.1 préparation du vrac

La production du virus rabique a été réalisée sur cellules Vero en utilisant un milieu d'infection virale sans sérum. Les cellules de la lignée Vero, ont été adaptées à des conditions de culture en milieu sans sérum comme décrit dans la demande WO 01/40443. Elles ont été ensuite transférées dans un biogénérateur contenant des microporteurs de cytodex 1 dans le milieu VP SFM (Invitrogen). Après une période de culture de 3 à 4 jours à 37°C en maintenant un pH d'environ 7.2 ± 0.2, une saturation en oxygène de 25 % ± 10 % et en soumettant le milieu à une faible agitation, les cellules ont été infectées avec du virus rabique (souche Pitmann- Moore) à une multiplicité d'infection de 0,01 en utilisant un milieu d'infection virale sans sérum contenant du milieu VP SFM (Invitrogen). On a récolté les surnageants de culture des cellules infectées aux jours J7 (R1), J11 (R2) et J15 (R3). Après chaque récolte, on a réintroduit du milieu d'infection virale neuf. Les surnageants de culture contenant le virus infectieux ont été clarifiés en utilisant deux filtrations successives : la première en utilisant un pré-filtre en polypropylène de 8 µm (Sartopure PP2, SARTORIUS) qui élimine les quelques microporteurs aspirés lors de la récolte, les cellules Vero décollées des supports et les débris cellulaires de taille importante ; la deuxième en utilisant un filtre PES, composé de la combinaison de deux filtres 0,8 µm et 0,45 µm (Sartopore 2, SARTORIUS) qui élimine les agrégats. La quantité de virus rabique présente dans les récoltes clarifiées a été déterminée sur la base de la mesure de la quantité de glycoprotéine G (gpG) effectuée par ELISA de la manière suivante :

On a répartit dans les puits d'une microplaque ELISA environ 0,12µg/100µl d'une solution d'un anticorps monoclonal 1112-1 anti-gpG (dont les caractéristiques sont décrites dans Journal of Clinical investigation (1989), volume 84, pages 971 à 975) préalablement diluée dans un tampon de «coating» (tampon carbonate/bicarbonate 0,2M, pH 9,6). Après une incubation d'une nuit en chambre froide, suivie de plusieurs lavages avec un tampon de lavage (tampon phosphate additionné de Tween 20, 0,05 %), on a distribué dans chaque puits 100 µl d'un tampon de saturation (tampon phosphate additionné de sérum albumine bovine à 1 %). Après une incubation d'une heure à 37°C, suivie de plusieurs lavages, on a réalisé une gamme de dilution de chaque échantillon à tester dans un tampon de dilution (tampon phosphate additionné de Tween 20 à 0,05 % et de sérum albumine à 0,1 %). En parallèle, on a réalisé dans chaque microplaque une gamme de dilution d'un étalon de référence, qui a été calibré vis-à-vis de la référence internationale du NIBSC (par exemple PISRAV). Après une nouvelle incubation d'une heure à 37°C, suivie de plusieurs lavages, on a distribué dans chaque puits 100 µl d'une solution d'un anticorps monoclonal D1 de souris anti-gpG (dont les caractéristiques sont décrites dans Biologicals (2003), volume 31, pages 9 à 16) qui a été biotinylé et utilisé après dilution au 1/5000 dans le tampon de dilution. Les plaques ont été laissées 1 heure à 37°C puis lavées à plusieurs reprises avant de distribuer dans chacun des puits 100 µl d'une solution de streptavidine couplée à la peroxydase (Southern Biotechnology Associates) préalablement diluée au 1/15000 dans le tampon de dilution. Après une nouvelle incubation d'une heure à 37°C suivie de plusieurs lavages on a distribué dans chaque puits 100 µl d'une solution d'un tampon citrate 0,05 M, pH 5 contenant le substrat de révélation (O-Phenylènediamine). Après un temps d'incubation de 30 minutes à température ambiante à l'abri de la lumière on a stoppé la réaction de révélation en ajoutant 50 µl/puits d'une solution d'H₂SO₄ 2N. La lecture spectrophotométrique des microplaques a été réalisée à deux longueurs d'onde (492 nm et 620 nm). La densité optique mesurée est la différence entre les deux lectures pour tenir compte de l'absorption du plastique. Le calcul de l'activité relative a été effectué par la méthode des droites parallèles selon les recommandations de la Pharmacopée Européenne. Le titre en virus rabique de l'échantillon est basé sur la détermination de la concentration en glycoprotéine G du virus rabique qui est exprimée en UI/ml par rapport à la référence.

Après avoir également contrôlé la conductivité et le pH du liquide clarifié on a déposé sur le support chromatographique Fractogel® EMD SO3⁻ (Merck) préalablement équilibré à l'aide d'un tampon Tris 20 mM, NaCl 150 mM, pH =7,5, l'équivalent de 50 UI environ de glycoprotéine G (mesuré par ELISA) par ml de support. Le support chromatographique a été ensuite lavé avec le tampon d'équilibration puis le virus rabique a été élué dans le tampon Tris 20mM, NaCl 600 mM, pH=7,5 avec récupération du pic viral en une fraction indépendante. On a procédé ensuite à une étape d'ultrafiltration sur membrane PES Medium Screen 100KD (PALL) suivie d'une diafiltration en tampon Tris 20 mM, NaCl 150 mM, pH = 7,5. On a rajouté une solution de MgCl₂ de sorte que la concentration dans le diafiltrat était 2 mM. Le traitement à la benzonase a été réalisé en ajoutant 15 U/ml de récolte brute au milieu réactionnel et en laissant le milieu réactionnel pendant 2 heures à température du laboratoire. L'étape d'ultracentrifugation a été réalisée sur coussins de sucrose 34-60 % avec un rotor 45 type Ti à 21000 rpm pendant 2 h à +5°C. Les fractions du gradient contenant le virus purifié ont été recueillies, rassemblées puis diluées dans le tampon phosphate 50 mM NaCl 150 mM, pH=7,5, de telle sorte que le volume final de la suspension de virus purifiés était environ 12,5 fois plus faible que le volume de la récolte clarifiée. La suspension de virus purifiés a été ensuite inactivée par un traitement à la β propiolactone suivie d'une inactivation de la β propiolactone par chauffage à une température d'environ 37°C pendant 2 heures. La préparation de virus purifiés et inactivés a été concentrée 6 fois par ultrafiltration sur membrane 10 kDa (Omega medium screen PALL) suivie d'une diafiltration en tampon phosphate 50 mM, NaCl 150 mM pH 7,5. La préparation de virus rabique obtenue est sous la forme d'un vrac concentré dont le titre basé sur la détermination de la concentration en glycoprotéine G était aux environ de 80 UI/ml. On a ensuite dialysé une aliquote du vrac contre l'un des 4 excipients (F01 à F04) dont les compositions étaient les suivantes.

### 1.2 Composition des excipients testés

F01 : tampon phosphate 50 mM, NaCl 150 mM, Maltose (5% p/p), pH=8,0
F02 : tampon phosphate 50 mM, Maltose (5% p/p), pH=8,0
F03 : tampon phosphate 50 mM, Maltose (5% p/p), poloxamère 188 (BASF) (0,001% p/p), pH=8,0
F04 : tampon 489 PM, pH=8,0 dont la composition est la suivante :

| Composants | Volume/Quantité |
|---|---|
| Acides Aminés essentiels | 40 ml |
| Acides Aminés non essentiels | 40 ml |
| Sorbitol (Roquette) | 20 g |
| Arginine chlorhydrate (Jerafrance) | 4 g |
| EDTA (Prolabo) | 0,148 g |
| Glutamate de sodium (SAFC) | 1,6 g |
| Na₂HPO₄, 2H₂0 | 8,428 g |
| NaHPO₄, 2H₂0 | 0,413 g |
| Maltose (Hayashibara) | 50 g |
| Urée | 4 g |
| Ajustement du pH à pH=8,0 avec de la soude 10 N | |
| Eau déminéralisée QSP | 1000ml |

La solution d'acides aminés essentiels a été préparée en dissolvant le contenu d'un flacon contenant 111,5g d'acides aminés essentiels (Gibco, Référence 074-90680N, lot numéro 14773) dans 5 litres d'eau pour préparation injectable acidifié par 100 ml d'HCl 12 N (Prolabo).

La solution d'acides aminés non essentiels a été préparée en dissolvant le contenu d'un flacon contenant 40,7g d'acides aminés non essentiels (Gibco, Référence 074-90681N, lot numéro 14775) dans 5 litres d'eau pour préparation injectable.

### 1.3 Essais effectués sur les 4 formulations du vrac

Le vrac a été formulé dans les 4 compositions d'excipients testés. La stabilité de ces quatre formulations (compositions vaccinales) a été évaluée au cours du temps à différentes température de conservation : -70°C, +5°C et à +37°C en mesurant à intervalles réguliers le titre en glycoprotéine G (exprimé en UI/ml). Les résultats obtenus sont représentés dans les tableaux I à III et sont exprimés en UI/ml.

**Tableau I : stabilité des 4 formulations du vrac à +37°C**

| Temps (semaines) | 0 | 1 | 2 |
|---|---|---|---|
| F01* | 41,25* | 30,21 | 17,51 |
| F02 | 60,41 | 23,15 | 5,12 |
| F03 | 60,29 | 23,16 | 5,2 |
| F04 | 57,26 | 52,37 | 52,68 |

**Tableau II : stabilité des 4 formulations du vrac à +5°C**

| Temps (semaines) | 0 | 3 | 12 | 24 |
|---|---|---|---|---|
| F01 | 41,25** | 39,71 | 45,56 | 38,55 |
| F02 | 60,41 | 53,29 | 42,27 | 38,92 |
| F03 | 60,29 | 52,64 | 47,75 | 41,33 |
| F04 | 57,26 | 62,19 | 61,06 | 61,32 |

**Tableau III : stabilité des 4 formulations du vrac à -70°C**

| Temps (semaines) | 0 | 3 | 12 | 24 |
|---|---|---|---|---|
| F01 | 41,25 | 33,81 | 37,61 | 27,59 |
| F02 | 60,41 | 53,52 | 53,06 | 53,62 |
| F03 | 60,29 | 57,87 | 60,15 | 56,46 |
| F04 | 57,26 | 57,48 | 59,92 | 56,56 |

| | | | | |
|---|---|---|---|---|
| * : F01, F02, F03 et F04 font référence à la composition des excipients dans lesquels ont été formulés le vrac. **: en UI/ml | | | | |

Ces résultats montrent que l'excipient F04 est celui qui stabilise le mieux la préparation de virus rabiques purifiés et inactivés en particulier lorsque la température de conservation augmente. Cela montre également qu'un excipient comprenant un sucre (maltose) et un surfactant non ionique tel que le poloxamère 188 dans une solution tamponnée à un pH de 8,0 ne suffit pas à stabiliser efficacement une préparation de virus rabique.

### Exemple 2 : Rôle du surfactant dans la stabilisation de compositions vaccinales contenant du virus rabique purifié

### 2.1 préparation des compositions vaccinales

Une suspension de virus rabiques purifiés a été obtenue en utilisant le même procédé que celui utilisé dans l'exemple 1. Après l'étape d'ultracentrifugation, la préparation de virus purifiée était sous une forme très concentrée. La concentration en protéines totales était de 7 mg/ml en utilisant la méthode de Bradford. Cette préparation de virus a été répartie dans 3 récipients en polypropylène puis diluée dans 3 excipients différents que l'on cherchait à évaluer pour leur rôle stabilisant, de sorte que la concentration finale en protéines totales dans chacun des excipients testés a été abaissée à 10µg/ml (facteur de dilution 1/700). La composition des 3 excipients testés ne différait que par leur contenu en poloxamère 188, mais contenait tous le tampon 489 PM, pH.=8,0 dont la composition est décrite dans l'exemple 1. L'excipient sans poloxamère 188 avait la même composition que l'excipient F04 décrit dans l'exemple 1. Les deux autres excipients ont été supplémentés respectivement avec 0,01 g/l de poloxamère 188 (excipient nommé F04 + 0,01% poloxamère) et avec 0,1 g/l de poloxamère 188 (excipient nommé F04 + 0,1% poloxamère).

### 2.2 Etudes de stabilité et résultats

La stabilité des 3 compositions vaccinales a été évaluée en les plaçant dans des conditions de conservation défavorables, c'est-à-dire en les soumettant à une succession de congélations et de décongélations selon le protocole suivant :
Les 3 compositions vaccinales ont été congelées à -70°C le même jour (Jour J0). Chacune des 3 compositions a été ensuite soumise à 3 décongélations à +5°C suivies d'une recongélation à -70°C dans les 7 à 14 heures suivant la décongélation aux jours 1, 2 et 3 (respectivement J1, J2 et J3). La stabilité des compositions vaccinales après chaque décongélation a été évaluée en mesurant la concentration protéique virale totale. La mesure a été effectuée en utilisant la technologie BIAcore qui repose sur l'utilisation de la résonnance plasmonique de surface. On a également analysé le profil de distribution de la taille des particules au cours des décongélations successives en utilisant la technique de diffusion quasi élastique de la lumière (Dynamic Light Scattering ou encore technologie DLS).

Pour mettre en œuvre la technologie BIAcore, on a dans un premier temps couplé par liaison de covalence l'anticorps monoclonal D1 de souris anti-gpG à une sensor chip en utilisant le kit de couplage fourni par le fabricant (Référence amine coupling kit, BR-1000-50). Ensuite, 30µl de l'échantillon à analyser a été automatiquement injecté dans l'appareil sur une période temps de 3 minutes. L'interaction du virus rabique présent dans l'échantillon avec l'anticorps monoclonal fixé sur la sensor chip a entrainé une modification de l'index de réfraction à la surface de la sensor chip se traduisant par une modification du signal enregistré (RU). Les résultats obtenus ont été enregistrés en unités arbitraires (ΔRU) et ont été comparés avec les résultats obtenus avec une gamme d'étalonnage d'une référence qui contenait une quantité connue de virus rabiques purifiés. Le ΔRU mesuré pour chaque échantillon a été converti en concentration protéique virale totale sur la base de la courbe d'étalonnage obtenue avec la référence. Pour chaque échantillon testé, les mesures ont été effectuées 4 fois (en quadruplicate). Après chaque mesure, la sensor chip a été nettoyée en effectuant deux injections successives de 10 µl d'un tampon Glycine (10 mM, pH=1,5). Les résultats obtenus pour chaque composition vaccinale testée sont représentés dans le tableau IV. Les valeurs sont exprimées en µg/ml de protéines virales.

**Table IV : Etude de la stabilité des 3 compositions vaccinales au cours d'un cycle de 3 décongélations et recongélations successives**

| Excipient stabilisant | D0 | D1 | D2 | D3 | Perte en titre viral |
|---|---|---|---|---|---|
| F04 | 11* (0.5)** | 3.4 (0.4) | 2.1 (0.3) | 1.4 (0.3) | 88% |
| F04 +0.001% poloxamère 188 | 14.8 (0.3) | 11.2 (0.5) | 10.8 (0.5) | 9.9 (0.6) | 33% |
| F04 + 0.01% Poloxamère 188 | 18.9 (0.0) | 15.7 (0.3) | 15.2 (0.4) | 14.7 (0.4) | 23% |

| | | | | | |
|---|---|---|---|---|---|
| * : titre moyen en protéines virales obtenu sur la base de 4 mesures réalisées sur l'échantillon testé en µg/ml ** : écart type calculé sur la base des 4 mesures réalisées sur l'échantillon testé est indiqué entre parenthèse. | | | | | |

Ces résultats montrent de façon claire que l'excipient F04 en l'absence de poloxamère est incapable de préserver de façon efficace la préparation de virus rabiques puisque la perte en titre viral est de 88% entre le titre à D0 et le titre mesuré après la troisième décongélation (D3). Par contre dès que l'excipient F04 est très légèrement supplémenté avec du poloxamère 188, la perte en titre viral est fortement diminué (il est de 33% quand F04 est supplémenté avec 0,001g/l de poloxamère 188 et de 23% quand F04 est supplémenté avec 0,01g/l de poloxamère 188. Ces résultats montrent que l'excipient doit contenir à la fois les composants qui sont présents dans le tampon 489 PM et du poloxamère 188 pour conserver efficacement une composition vaccinale contenant du virus rabique.

Le profil de distribution de la taille des particules de chacune des 3 compositions vaccinales a été aussi analysé après chaque décongélation par DLS en utilisant l'appareil Zetasizer Nano Zs (Malvern Instrument). On a introduit dans une cuvette à usage unique en polystyrène 450µl de l'échantillon à analyser que l'on a soumis ensuite à une radiation laser monochromatique (laser HeNe, λ=632,8 nm). Le signal enregistré par l'appareil correspondait aux fluctuations de la lumière dispersée résultant du mouvement Brownien des particules. Les données enregistrées ont été traitées par un logiciel. Les résultats ont été finalement représentés sous la forme d'une courbe de distribution de la taille des particules pour chaque échantillon testé. Les 2 figures représentent une superposition des 4 courbes de distribution de la taille des particules qui ont été obtenues respectivement avec une composition de virus rabiques stabilisée avec l'excipient F04 +0.001% poloxamère 188 aux jours J0 (avant congélation), J1, J2, et J3 (Figure 1), et avec une composition de virus rabiques stabilisée avec l'excipient F04 +0.01% poloxamère 188 (Figure 2).

Les deux figures montrent clairement que :
1) les courbes de distribution de la taille des particules sont unimodales et ont un profil gaussien dont la médiane se situe aux environs de 180 nm. Cela signifie que la population de particules virales dans la composition vaccinale stabilisée avec l'excipient F04 +0.001% poloxamère 188 ou l'excipient F04 +0.01% poloxamère 188 est homogène, ne contient pas d'agrégats et comprend une population de virus rabiques entiers similaire à la population de virus rabiques sauvages et,
2) les courbes de distribution de la taille des particules ne sont pas modifiées quand les compositions vaccinales de virus rabiques sont soumises à plusieurs décongélations et recongélations successives.

La présence d'un tensioactif non ionique tel que le poloxamère 188 dans la composition de l'excipient préserve également l'intégrité physique de la population de virus rabiques même lorsque la composition vaccinale est placée dans de mauvaises conditions de conservation.

### 2.3 Etudes de stabilité sur des compositions vaccinales lyophilisées

### 2.3.1 : Préparation des compositions vaccinale lyophilisées

Un vrac a d'abord été préparé à partir d'une préparation de virus purifiés entiers inactivés qui a été obtenue en utilisant le procédé décrit dans l'exemple 1. Après l'étape d'inactivation virale, la préparation de virus rabiques purifiés et inactivés a été diafiltrée sur une membrane de 10KDa (Omega medium screen PALL) en utilisant un excipient appelé 488 TM qui a été préparé à partir d'un milieu 488 dont la composition est la suivante (voir tableau ci dessous) :

| Composants | Volume/Quantité |
|---|---|
| Mélange d'Acides Aminés essentiels et d'acides aminés non essentiels | 200 ml |
| Sorbitol (Roquette) | 50 g |
| Arginine chlorhydrate (Jerafrance) | 10 g |
| EDTA (Prolabo) | 0,37 g |
| Glutamate de sodium (SAFC) | 4 g |
| Urée | 10 g |
| Tris Na H₂PO4, 2H₂0 (SAFC) | 2,42g |
| Eau déminéralisée QSP | 1000ml |
| Acide chlorhydrique 6N (Sanofi) QSP | pH=8,0 |

Le mélange d'acides aminés essentiels et non essentiels a été préparé en mélangeant 2,5 litres de la solution d'acides aminés essentiels préparée selon l'exemple 1 avec 2,5 litres de la solution d'acides aminés non essentiels préparée selon l'exemple 1 et en ajustant le pH à environ 7,2 au moyen d'une solution de soude à 30%.

La composition de l'excipient 488 TM est la suivante :

| Composants | Volume/Quantité |
|---|---|
| Milieu 488 | 400 ml |
| Maltose (Hayashibara) | 50 g |
| Tris Na H₂PO4, 2H₂0 (SAFC) | 1.45g |
| Eau déminéralisée QSP | 1000ml |
| Acide chlorhydrique 6N (sanofi) QSP | pH=8,0 |

La préparation vaccinale obtenue était sous la forme d'un vrac dans l'excipient 488 TM dont le titre viral basé sur la détermination par ELISA de la concentration en glycoprotéine G était aux environs de 12 UI/ml. Le vrac a ensuite été conservé sous forme congelée à -70°C jusqu'au moment de la préparation des compositions vaccinales lyophilisées.

Juste avant l'étape de lyophilisation, une partie du vrac a été décongelée et diluée pour ramener le titre final en glycoprotéine G à environ 8 UI/ml en utilisant :
- soit l'excipient F'04 dont la composition contient 1 volume de l'excipient 488 TM pour 3 volumes de tampon phopshate 50 mM, la concentration en maltose étant ajustée à 50g/l, pH=8,0
- soit l'excipient F'05 qui est identique à l'excipient F'04 mais auquel on a ajouté du poloxamère 188 à une concentration finale de 0,01g/l.

On a ensuite répartit les deux compositions vaccinales dans des flacons de lyophilisation à raison de 0,4 ml/flacon puis on a réalisé un cycle de lyophilisation classique comprenant une phase de congélation à une température <-40°C suivie de deux phases de dessiccation successives, la première phase de dessiccation étant réalisée à une température d'environ -15°C sous une pression d'environ 80 µbar et la deuxième à une température d'environ +40°C sous une pression d'environ 80 µbar.

Chaque flacon de lyophilisation contenait une dose de la composition vaccinale à tester. Les compositions vaccinales lyophilisées obtenues différaient seulement par la présence ou non du surfactant selon que le vrac a été dilué dans l'excipient F'04 ou F'05.

On a indiqué ci dessous la composition de l'excipient contenu dans une dose de la composition vaccinale sous forme lyophilisée préparée à partir de l'excipient F'05.

| Composant | Quantité en mg/dose |
|---|---|
| Mélange d'acides aminés essentiels et non essentiels | 0,12 |
| Poloxamère 188 | 0,004 |
| Maltose | 20,00 |
| Chlorhydrate d'Arginine | 0,41 |
| Sorbitol | 2,05 |
| EDTA disodique | 0,01 |
| Urée | 0,41 |

| | |
|---|---|
| Glutamate de sodium | 0,16 |
| Na2HPO4, 2H20 | 2,53 |
| NaH2PO4, 2H20 | 0,12 |
| Tris | 0,24 |
| HCl | QSP |
| NaOH | QSP |

### 2 .3. 2 : Efficacité des compositions vaccinales lyophilisées

Les deux compositions vaccinales lyophilisées qui ont été préparées en utilisant deux excipients stabilisants différents ont été ensuite testées pour leur efficacité en utilisant le test officiel NIH. On a analysé « l'efficacité » des deux compositions vaccinales lyophilisées, soit immédiatement après la lyophilisation (J0), soit après conservation d'une semaine à 45°C, soit également après conservation d'un mois à 37°C. On a utilisé le seul test d'activité officiellement reconnu, le test NIH, qui permet de déterminer le nombre d'UIs contenues dans chacune des doses des compositions vaccinales testées. Si le nombre d'UIs est ≥2,5 UI la dose de vaccin testée est efficace. Le test NIH a été effectué à chaque fois en « double » pour chacune des conditions testées en prélevant au hasard deux flacons lyophilisés dans le lot de flacons préparés et conservés dans les mêmes conditions et en effectuant sur ces prélèvements un test NIH. Le protocole utilisé correspond à la monographie 0216 de la Pharmacopée Européenne. Ce test est un test d'épreuve réalisé chez la souris, basé sur la comparaison de la protection conférée par la dose de vaccin testé avec celle conférée par une quantité connue d'un vaccin antirabique de référence. Le vaccin antirabique de référence est calibré en unités internationales (UI). L'unité internationale (UI) est l'activité contenue dans une quantité déterminée d'un standard international. L'équivalence en UI du standard international a été établie par l'OMS. Après avoir vérifié que les paramètres de contrôle du test d'efficacité étaient bien respectés, on a déterminé à partir des valeurs de la dose protectrice 50% obtenue pour chaque prélèvement testé et pour la préparation de référence (calibré en UIs), le nombre d'UIs qui sont contenues dans la dose de vaccin testé.

Les résultats obtenus sont représentés dans le tableau V ci-dessous

**Tableau V : efficacité des compositions vaccinales lyophilisées en fonction de la composition de l'excipient de lyophilisation utilisé et des conditions de conservation.**

| Durée (en jours) et température de conservation de la composition lyophilisée | J0/ + 4°C | J7/ +45°C | J30/ +37°C |
|---|---|---|---|
| F'04 | 0,8* (0,4 ; 1,8)** | 0,9 (0,4; 2,1) | NT |
| F'05 | 6,1 (1,6; 41,3) | 2,8 (0,9 ; 7,9) | 3,5 (1,4 ; 8,7) |

| | | | |
|---|---|---|---|
| *: valeur moyenne exprimée en UI obtenues (tous les groupes de souris qui ont été éprouvés contiennent 16 souris) ** : représente les valeurs extrêmes obtenues exprimées en UI | | | |

### NT : Non testé

Ces résultats sont en accord avec les résultats discutés dans le paragraphe précédent (2.2) et montrent que du poloxamère 188 doit être inclus dans la composition de l'excipient utilisé pour conserver les compositions vaccinales lyophilisées. En effet, seules les compositions vaccinales lyophilisées qui renferment l'excipent F'05 (qui contient du poloxamère 188) contiennent une dose efficace de vaccin puisque la valeur moyenne en UI est supérieure à 2,5 UI. Ces compositions vaccinales restent stables puisque leur efficacité n'est pas diminuée au cours du temps dans les conditions de conservation qui ont été testées.

Pour résumer, les résultats qui sont présentés dans l'exemple 2, montrent que les compositions vaccinales renfermant du virus rabique peuvent être bien conservées avec un excipient stabilisant dont la composition comprend un tampon phosphate un mélange d'acides aminés essentiels et non essentiels, un sucre tel que le maltose, un polyol tel que le sorbitol, de l'EDTA, de l'urée en combinaison avec un surfactant non ionique tel que le poloxamère 188. De plus et de façon intéressante, «l'effet stabilisant» du poloxamère 188 s'exerce à une très faible concentration qui est trop basse pour que le poloxamère 188 puisse activer le système immunitaire ou agir comme adjuvant de la réponse immune.

### Exemple 3 : étude de la stabilité de compositions vaccinales contenant du virus rabique purifié et inactivé

Les exemples 1 et 2 ayant montré l'importance de combiner les composants d'un excipient qui comprend un mélange d'acides aminés essentiels et non essentiels , du maltose, du sorbitol, de l'EDTA, de l'urée dans un tampon à base de phosphate et/ou de Tris avec du poloxamère 188, le but des études présentées ici était de confirmer que cette combinaison stabilisait efficacement des compositions vaccinales de virus rabiques sous différentes formes (liquide, congelée ou lyophilisée) dans une large gamme de température de conservation (+37°C, +25°C, +5°C, -70°C) et sur une longue période de temps. Ces études ont été réalisées sur plusieurs compositions vaccinales de virus rabiques qui ont été conservés sous la forme d'un vrac congelé à une température ≤-35°C, sous la forme d'un produit final vrac liquide à +5°C ou sous la forme de doses unitaires lyophilisées conservées à +5°C, +25°C ou + 37°C.

### 3.1 Etudes de la stabilité de lots de vaccins antirabiques sous la forme de vrac congelés.

Le virus rabique servant à la préparation des lots de vrac a été préparé selon le procédé tel que décrit dans l'exemple 1. Après l'étape d'inactivation virale, la préparation de virus purifiée et inactivée a été diafiltrée sur une membrane de 10KDa en utilisant comme tampon de diafiltration un tampon phosphate 50 mM. Dans un deuxième temps on a mélangé 1 volume de rétentat avec 1 volume d'excipient de stabilisation dont la composition est celle du tampon 489 PM décrit dans l'exemple 1, (hormis le fait que la concentration de chacun des composants est deux fois plus importantes) auquel on a ajouté du poloxamère 188 à la concentration de 0,02 g/l, pH ≈ 8,0. On a obtenu un vrac ayant une concentration en protéines totales de 50 µg/ml parmi lesquelles plus de 70% sont des protéines du virus rabique et qui contient moins de 100 pg/ml d'ADN résiduel. On a étudié la stabilité du vrac au cours du temps après conservation à -35°C et à -70°C en mesurant tous les 3 mois le titre en glycoprotéine G. Les résultats obtenus sont présentés dans le tableau VI ci-dessous.

**Tableau VI : Stabilité d'un lot de vrac conservé à -35°C ou à -70°C**

| Température de conservation | T0 | T0 + 3 mois | T0 + 6 mois | T0 + 9 mois | T0 + 12 mois |
|---|---|---|---|---|---|
| - 35°C | 20,4* | 20,84 | 24,4 | 24,3 | 22,4 |
| - 70°C | 21,3 | NT | 22,4 | 22,8 | 21,8 |

| | | | | | |
|---|---|---|---|---|---|
| *: titre exprimé en UI/ml NT : non testé | | | | | |

Après 12 mois de stockage à -35°C ou à -70°C on n'observe pas de dégradation de la glycoprotéine G qui est l'antigène majeur du virus rabique ce qui confirme la bonne stabilité de la composition vaccinale sous forme de vrac congelé. De plus, l'analyse de la distribution de la taille des particules virales par DLS après une conservation de 12 mois à -35°C ou à -70°C ont montré que les profils conservaient une allure gaussienne centrée sur une valeur médiane de 180 nm (profils similaires à ceux des figures 1 et 2). Il n'y a pas de morcellement des profils indiquant la présence d'agrégats viraux. Les résultats ont montré également que la population de particules de virus rabiques ne se modifiait pas au cours du temps. On n'observe pas de dégradation ni d'agrégation des particules virales au cours du temps.

### 3.2 : Etudes de la stabilité de lots de vaccins antirabiques sous la forme de produit final vrac (PFV) liquide.

On a préparé 3 lots de vaccins sous la forme de PFV liquide à partir de 3 lots de vrac après dilution dans le tampon 489 PM (composition décrite dans l'exemple 1) auquel on ajouté du poloxamère 188 à une concentration finale de 0,01g/l, pH≈ 8,0. Ils contenaient moins de 100 pg/ml d'ADN résiduel (mesuré par PCR quantitative). La concentration en protéines totales était d'environ 15 µg/ml et plus de 70% des protéines totales étaient représentées par des protéines virales après analyse densitométrique de l'électrophorèse sur gel de polyacrylamide. La stabilité des lots de PFV conservés à +5°C et à +25°C a été étudié en mesurant à intervalles réguliers le taux de glycoprotéine G par ELISA sur une période de temps de 3 mois pour les lots de PFV conservés à +5°C et sur période de temps de 30 jours pour les lots de PFV conservés à +25°C. Les résultats obtenus exprimés en UI/ml sont rassemblés dans les tableaux VII et VIII ci-après :

**Table VII: Stabilité des 3 lots de PFV conservés à +5°C**

| N° de lot | T0 | T0+ 1 mois | T0 + 2 mois | T0 + 3 mois |
|---|---|---|---|---|
| N°1 | 10.5 | 12.0 | 8.6 | 10.8 |
| N°2 | 11.1 | 10.7 | 9.3 | 11.9 |
| N°3 | 13.9 | 11.1 | 11.6 | 11.2 |

**Table VIII: Stabilité des 3 lots de PFV conservés à +25°C**

| N° de lot | T0 | T0 +15 jours | T0 +30 jours |
|---|---|---|---|
| N°1 | 12.0 | 10.9 | 10.9 |
| N°2 | 10.7 | 11.4 | 10.1 |
| N°3 | 11.1 | 12.0 | 11.5 |

Ces résultats montrent qu'il n'y a pas de dégradation de la glycoprotéine G au cours de la conservation des lots de PFV sous forme liquide à +5°C et à +25°C pendant les périodes de temps étudiés. Cela confirme que les vaccins antirabiques peuvent être conservés sous forme liquide au froid (+5°C) pendant au moins 3 mois et à température ambiante (+25°C) pendant au moins 30 jours sans observer de dégradation du virus.

### 3.3 : Etudes de la stabilité de lots de vaccins antirabiques sous forme lyophilisée

2 lots de vaccins antirabiques sous forme lyophilisée (LL1 et LL2) ont été préparés à partir des lots de PFV de l'exemple 3.2 qui ont été répartis dans des flacons de lyophilisation en verre à raison de 0,3 ml/flacon avant de procéder à un cycle de lyophilisation au cours duquel la congélation a été réalisée à une température ≤-40°C, la phase de dessiccation primaire à une température voisine de -15°C sous une pression d'environ 80 µbar jusqu'à sublimation complète de la glace et la phase de dessication secondaire à une température voisine de +40°C sous une pression d'environ 80 µbar jusqu'à ce que le taux d'humidité résiduel soit <3% dans les lyophilisats. Les pertes en titre viral lors de l'étape de lyophilisation ont été <5%.

La composition de l'excipient contenu dans une dose vaccinale sous forme lyophilisée est la suivante :

| Composant | Quantité en mg/dose |
|---|---|
| Mélange d'acides aminés essentiels et non essentiels | 0,37 |
| Poloxamère 188 | 0,003 |
| Maltose | 15,00 |
| Chlorhydrate d'Arginine | 1,20 |
| Sorbitol | 6,00 |
| EDTA disodique | 0,04 |
| Urée | 1,2 |
| Glutamate de sodium | 0,15 |
| Na2HPO4, 2H20 | 2,53 |
| NaH2PO4, 2H20 | 0,12 |
| HCl | QSP |
| NaOH | QSP |

Les doses unitaires lyophilisées obtenues avaient un aspect homogène blanc. Le pH de la suspension de virus rabiques après reprise des lyophilisats par 0,5 ml d'une solution de chlorure de sodium à 0,4% se situait dans une gamme de 7 ,8 ± 0,5. On a étudié la stabilité des deux lots lyophilisées au cours du temps à 3 températures de conservation : 35-39°C, 23-27°C et 2-8°C en vérifiant l'aspect des lyophilisats avant et après reconstitution, en mesurant l'humidité résiduelle, en contrôlant le pH, le titre viral, et en testant l'efficacité des doses lyophilisées dans le test NIH. La première dilution des gammes de dilution qui ont été effectuées pour tester les doses lyophilisées dans le test NIH après reprise dans le chlorure de sodium a été réalisée dans une solution de chlorure de sodium à 0,9% contenant 2g/l d'albumine humaine.

Les titres viraux ont été mesurés sur la base du taux de glycoprotéine G par ELISA et exprimés en UI/ml.

Les résultats des tests concernant l'aspect des lyophilisats et de mesure de l'humidité résiduelle et de pH étaient tous conformes aux spécificités (c'est-à-dire un aspect blanc homogène pour le lyophilisât, une solution limpide incolore après reconstitution en sérum physiologique hypotonique et un taux d'humidité résiduelle <3%) quelle que soit la température et la durée de conservation testée. Le pH est resté stable dans une gamme de 7 ,8 ± 0,5 pendant toute la durée des études de stabilité. Les résultats concernant les titres viraux et des tests d'efficacité (NIH) réalisés sur les doses unitaires sont rassemblés dans les tableaux IX à XI ci dessous

**Tableau IX : stabilité des deux lots de vaccins antirabiques conservés sous forme lyophilisée à +5°C**

| lot | Test | T0 | 1M | 3 M | 6M | 9M | 12 M | 18 M |
|---|---|---|---|---|---|---|---|---|
| LL1 | Titre viral | 7.1* | 7.7 | 7.8 | 7.9 | 7.3 | 8.8 | 7.8 |
| | NIH | 3.4** (1.9-6.1) | ND | ND | 6.3 (3.6-10.9) | ND | 6.0 (3.5-10.3) | 6.3 (33-11.9) |
| LL2 | Titre viral | 6.1 | 6.6 | 6.7 | 6.5 | 6.9 | 6.4 | ND |
| | NIH | 4.7 (2.6-8.6) | ND | ND | 7.9 (4.5-14) | ND | 6.7 (3.7-12.) | ND |

**Tableau X : stabilité des deux lots de vaccins antirabiques conservés sous forme lyophilisée à +25°C**

| Lot | Test | T0 | 1M | 3 M | 6M |
|---|---|---|---|---|---|
| LL1 | Titre viral | 7.7 | 7.1 | 7.8 | 6.9 |
| | NIH | ND | ND | ND | 7.0 (4.0- 12.2) |
| LL2 | Titre viral | 6.6 | 6.1 | 6.0 | 6.2 |
| | NIH | ND | 6.8 (3.9- 11.8) | ND | 8.9 (5.1-15.6) |

**Tableau XI : stabilité des deux lots de vaccins antirabiques conservés sous forme lyophilisée à +37°C**

| Lot | Test | T0 | 1M | 3 M | 6M |
|---|---|---|---|---|---|
| LL1 | Titre viral | 7.7 | 7.5 | 10.1 | 10.3 |
| | NIH | ND | 7.1 (3.1-16.1) | ND | 8.6 (5.0-14.9) |
| LL2 | Titre viral | 6.6 | 7.2 | 7.5 | 7.7 |
| | NIH | ND | 8.8 (3.7-20.9) | ND | 6.2 (3.5-11.3) |

| | | | | | |
|---|---|---|---|---|---|
| M : signifie mois ND : signifie non déterminé * : valeur exprimée en UI/ml ** : valeur moyenne exprimée en nombre d'UI complétée par les valeurs extrêmes observées indiquées entre parenthèse. | | | | | |

L'ensemble de ces résultats confirment que les vaccins antirabiques conservés sous forme lyophilisée restent très stables et ne perdent pas en efficacité au cours du temps dans les conditions de température testées. Les titres en glycoprotéines G et les valeurs du test NIH (nettement supérieures à 2,5 UI) restent stables au cours du temps.

En conclusion, tous les tests de stabilité mis en œuvre sur des lots de vaccins antirabiques conservés sous forme congelée, sous forme liquide ou sous forme lyophilisée montrent que ces lots restent stables au cours du temps dans les conditions de température testées. Cela confirme qu'un excipient dont la composition comprend un tampon, un mélange d'acides aminés essentiels et non essentiels, du maltose, du sorbitol, de l'EDTA, de l'urée et du poloxamère 188 stabilise efficacement une composition vaccinale antirabique quelle que soit sa présentation (congelée, liquide ou lyophilisée) et dans une large gamme de températures de conservation.

## Revendications

1. Une composition vaccinale comprenant :
a) Une préparation de virus rabiques entiers inactivés et,
b) un excipient stabilisant qui comprend :
i. une solution tampon,
ii. un mélange d'acides aminés essentiels et non essentiels,
iii. un disaccharide,
iv. un polyol,
v. un agent chélatant,
vi. de l'urée ou un dérivé de l'urée, et
vii. un tensioactif non ionique.

2. Une composition selon la revendication 1, **caractérisée en ce que** le mélange d'acides aminés essentiels et non essentiels comprend au moins de l'arginine ou un sel d'arginine et de l'acide glutamique ou un sel d'acide glutamique.

3. Une composition selon l'une des revendications 1 à 2, **caractérisée en ce que** le disaccharide est le maltose.

4. Une composition selon l'une des revendications 1 à 3, **caractérisée en ce que** le polyol est le sorbitol.

5. Une composition selon l'une des revendications 1 à 4, **caractérisée en ce que** l'agent chélatant est l'EDTA ou un sel d'EDTA.

6. Une composition selon l'une des revendications 1 à 5, **caractérisée en ce que** le tensioactif non ionique est un poloxamère.

7. Une composition selon l'une des revendications 1 à 6, **caractérisée en ce que** le tensioactif non ionique est le poloxamère 188.

8. Une composition selon l'une des revendications 1 à 7, **caractérisée en ce que** la quantité d'acides aminés essentiels et non essentiels contenue dans une dose efficace de la composition vaccinale est comprise entre 0,5 et 2,5 mg ; et/ou la quantité totale de disaccharide et de polyol contenue dans une dose efficace de la composition vaccinale est comprise entre 10 et 50 mg ; et/ou la quantité d'agent chélatant contenue dans une dose efficace de la composition vaccinale est comprise entre 0,01 et 0,1 mg ; et/ou la quantité d'urée ou de dérivé de l'urée contenue dans une dose efficace de la composition vaccinale est comprise entre 0,3 et 1,5 mg ; et/ou la quantité de tensioactif non ionique contenue dans une dose efficace de la composition vaccinale est comprise entre 0,001 et 0,5 mg.

9. Une composition selon l'une des revendications 1 à 8, **caractérisée en ce que** le pH de la composition vaccinale est compris entre 7,0 et 10,0 et de préférence entre 7,0 et 9,0.

10. Une composition selon l'une des revendications 1 à 9, **caractérisée en ce que** la solution tampon est un tampon phosphate, un tampon Tris, un tampon HEPES ou un mélange de ceux-ci.

11. Une composition selon l'une des revendications 1 à 10, **caractérisée en ce que** la solution tampon est un tampon phosphate dont la molarité est comprise entre 10 et 100 mM.

12. Un procédé de préparation d'une composition vaccinale contenant une préparation de virus rabiques entiers purifiés et inactivés, selon lequel:
a. on produit une préparation de virus rabiques entiers en récoltant le surnageant d'une culture de cellules infectées par du virus,
b. on purifie et on inactive la préparation de virus rabiques entiers ou alternativement on inactive puis on purifie la préparation de virus entiers, et
c. on dilue la préparation de virus rabiques entiers purifiés et inactivés dans un excipient stabilisant dont la composition comprend :
i. une solution tampon,
ii. un mélange d'acides aminés essentiels et non essentiels,
iii. un disaccharide,
iv. un polyol,
v. un agent chélatant,
vi. de l'urée ou un dérivé de l'urée, et
vii. un tensioactif non ionique.

13. Un procédé selon la revendication 12, selon lequel après avoir dilué la préparation de virus rabiques entiers purifiés et inactivés, on répartit la composition vaccinale ainsi obtenue dans des dispositifs de conditionnement, et optionnellement on lyophilise la composition vaccinale.

14. Une composition vaccinale contenant une préparation de virus rabiques entiers purifiés et inactivés sous une forme lyophilisée obtenue selon le procédé de la revendication 13.

15. Un excipient stabilisant pour un vaccin à virus rabiques entiers inactivés dont la composition comprend :
i. une solution tampon,
ii. un mélange d'acides aminés essentiels et non essentiels,
iii. un disaccharide,
iv. un polyol,
v. un agent chélatant,
vi. de l'urée ou un dérivé de l'urée, et
vii. un tensioactif non ionique.
